# EUROPEAN PATENT SPECIFICATION

(11) **EP 1 845 841 B1**
(45) Date of publication and mention of the grant of the patent: **09.10.2013**
(21) Application number: 05820467.8
(22) Date of filing: 28.12.2005
(51) Int. Cl.: A61M 16/04, A61M 16/08, A61M 16/10, A61B 5/097

(54) **CAPNOGRAPHIC SAMPLING CATHETER**
CAPNOGRAFISCHER PROBENAHMEKATHETER
CATHETER D'ECHANTILLONNAGE CAPNOGRAPHIQUE

(30) Priority: 28.12.2004 US 640962 P
(43) Date of publication of application: 24.10.2007
(73) Proprietor: Oridion Medical 1987 Ltd., 91450 Jerusalem (IL)
(72) Inventor: LEVITSKY, Gershon, 97792 Jerusalem (IL); COLMAN, Joshua, Lewis, 97861 Jerusalem (IL)
(74) Representative: Miller, James Lionel Woolverton
(86) International application number: PCT/IL2005/001391
(87) International publication number: WO 2006/070366

(56) References cited:
- WO-A1-89/09565
- WO-A1-03/068301
- US-A- 3 658 053
- US-A- 5 046 491
- US-A- 5 555 890
- US-A- 6 159 158
- US-A1- 2001 031 929

## Description

### FIELD OF THE INVENTION

The present invention relates to the field of catheters for use in sampling the breath of subjects, especially for the purpose of providing capnographic data concerning the subject.

### BACKGROUND OF THE INVENTION

The following U.S. Patents are believed to represent state of the art: 5,787,885; 5,383,469; 5,335,656 and 4,485,822.

US-A-5046491 discloses a hollow flexible oral gas capture cylinder connected to a nasal gas annular member by a connected stem and located near the patient's facial surfaces between the patient's upper lips and nares.

US2001/0031929-A discloses a pair of nasal tubes extending approximately 1 cm to collect expired nasal air. A pair of tubes, each with a larger diameter and a larger length, e.g. 1.5cm, collect expired oral gases. The tubes are housed in a body which has a critical curvature to conform to the facial structure of the patient.

US-A-5555890 discloses a nasogastric tube having a sampling portion with a plurality of sampling ports.

### SUMMARY OF THE INVENTION

The present invention seeks to provide a sampling catheter for use with a capnographic system. There is thus provided in accordance with a preferred embodiment of the present invention a system for analyzing the exhaled breath of a subject, including a gas analyzer, sensitive to at least one component of the exhaled breath and a gas sampling catheter having a first end and a second end, the first end being in fluid flow communication with the gas analyzer, the gas sampling catheter has a plurality of exhaled breath sampling holes formed therein in the vicinity of said second end.

In accordance with a preferred embodiment of the present invention different ones of the plurality of sampling holes are disposed at different longitudinal positions along the gas sampling catheter. Additionally or alternatively, different ones of the plurality of sampling holes are disposed at different circumferential positions around the gas sampling catheter

In accordance with a preferred embodiment of the present invention the system also includes a suction element, configured to remove fluid from the gas sampling catheter.

In accordance with another preferred embodiment of the present invention the plurality of sampling holes are disposed in a generally helical pattern.

In accordance with yet another preferred embodiment of the present invention the second end of the gas sampling catheter is sealed. Preferably, the first end of the gas sampling catheter is directly connected to the gas analyzer, thereby forming the fluid flow communication therebetween. Alternatively, the first end of the gas sampling catheter is connected to the gas analyzer by means of a breath conduit.

In accordance with a further preferred embodiment of the present invention the gas sampling catheter is fitted with a suction port, the suction port being adapted to connect to the suction element.

In accordance with a still further preferred embodiment of the present invention the gas sampling catheter has at least one circumferential protrusion formed therearound.

In accordance with yet a further preferred embodiment of the present invention the system also includes an oxygen delivery tube mounted onto the gas sampling catheter, thereby forming a multi-lumen tube therewith and an oxygen source connected to the oxygen delivery tube for supply of oxygen thereto.

There is additionally provided in accordance with a further preferred embodiment of the present invention a gas sampling catheter including a catheter tube being formed with a plurality of exhaled breath sampling holes.

In accordance with a preferred embodiment of the present invention different ones of the plurality of sampling holes are disposed at different longitudinal positions along the catheter tube. Additionally or alternatively, different ones of the plurality of sampling holes are disposed at different circumferential positions around the catheter tube

In accordance with a preferred embodiment of the present invention the plurality of sampling holes are disposed in a generally helical pattern.

In accordance with another preferred embodiment of the present invention one end of the catheter tube is sealed. Preferably, the catheter tube has at least one circumferential protrusion formed therearound.

### BRIEF DESCRIPTION OF THE DRAWINGS

The present invention will be understood and appreciated more fully from the following detailed description, taken in conjunction with the drawings in which:
Fig. 1 is a schematic illustration of a sampling catheter constructed and operative in accordance with a preferred embodiment of the present invention, which is typically used in a capnographic system;
Fig. 2 is a schematic illustration of a sampling catheter constructed and operative in accordance with another preferred embodiment of the present invention, which is typically used in a capnographic system;
Figs. 3A and 3B are sectional illustrations taken along respective section lines IIIA-IIIA and IIIB-IIIB in Fig. 2; and
Fig. 4 is a schematic illustration of a sampling catheter constructed and operative in accordance with yet another preferred embodiment of the present invention, which is typically used in a capnographic system.

### DETAILED DESCRIPTION OF PREFERRED EMBODIMENTS

Catheters are thin flexible tubes, generally open at both ends, which are widely used in health care, for insertion into a bodily cavity, duct or vessel, generally for either supplying or extracting fluids from organs accessible through the bodily cavity or duct.

Traditional breath sampling is generally performed at the oral or nasal orifices using an appropriate cannula. There are a number of problems with such nasal or nasal/oral breath sampling such as dilution of the breath sample by the ambient air, and the difficulty of sampling effectively from a patient that alternates between nasal and oral breathing.

Breath sampling by means of a catheter is more accurate than sampling methods at the oral or nasal orifices using an appropriate cannula, since catheter sampling is performed at the oral/nasal junction, or even closer to the source of the breath, and hence well away from any diluent effects from the sampling orifice. However, when using a breath sampling catheter, blockage of the sampling line by entrance thereto of liquids from the oral/nasal junction may occur. The design and structure of the sampling catheter of the present invention ensure that little or no blockage will occur, as described with more detail hereinbelow.

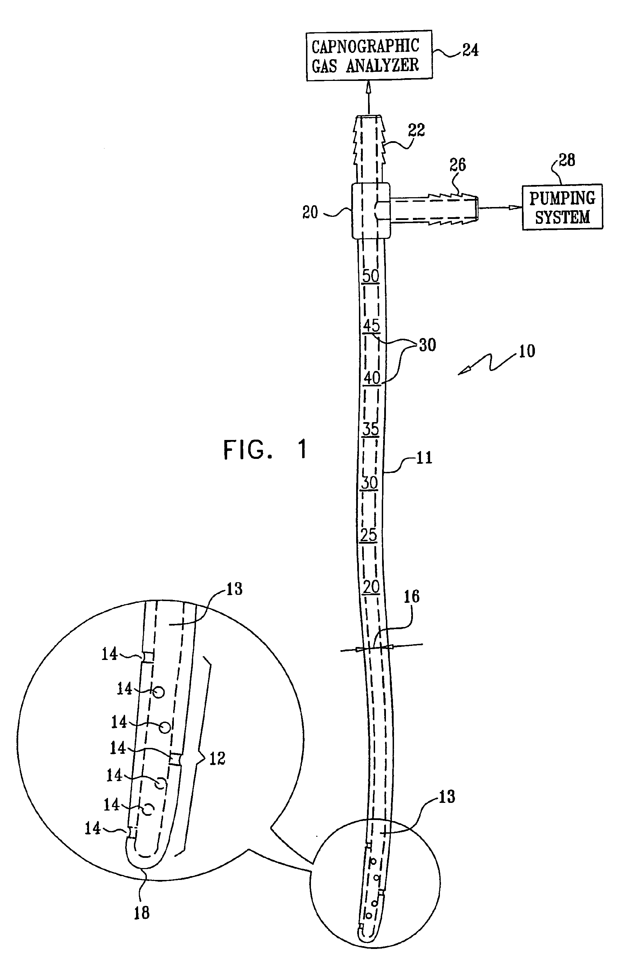

Reference is now made to Fig. 1, which is a schematic illustration of a capnographic sampling system constructed and operative in accordance with a preferred embodiment of the present invention. Capnographic system 10 includes a sampling catheter 11 having a sampling portion 12, which is preferably located at a lower end 13 of sampling catheter 11. A number of sampling holes 14 are formed in the wall of the sampling catheter 11, preferably within the sampling portion 12. Sampling catheter 11 additionally includes an internal bore 16, forming a passageway for the sampled fluid.

Sampling holes 14 are preferably arranged at different longitudinal positions along the length of sampling catheter 11 and at different circumferential positions therearound. A preferred pattern for the location of the sampling holes 14 is in the form of a helix. This structure ensures that at least some of the sampling holes 14 remain open, even when one side of the catheter circumference is in contact with the passageway into which the sampling catheter 11 has been inserted.

Additionally, the use of several sampling holes 14 ensures that even if a few of the sampling holes 14 are in contact with liquids, the gas sample will continue to flow through the open holes. This is due to the fact that the relatively small sampling holes have a greater resistance to liquids than to the sampled breath. Preferably, the gas sampling catheter 11 is formed of a hydrophobic material, and is therefore less prone to blockage of the sampling holes 14.

The diameter of the sampling holes 14 and the longitudinal distance between them are preferably determined according to the width of internal bore 16 and to the flow rate within the passageway through which the sample is taken. According to one preferred configuration of the sampling catheter 11, five sampling holes 14, each of a diameter in the range of 0.3 - 0.8mm are disposed within a 4cm-long sampling portion 12. Alternatively, a longer sampling portion 12 may be provided.

The sampling catheter 11 preferably has a narrow overall diameter and a narrow internal bore 16. For adult-sized use, the internal bore 16 is preferably of the order of 1.0mm, and for pediatric use, even less. The use of a catheter with such a narrow diameter is advantageous as it is readily filled by the sampled breath, creating a faster response time. Furthermore, it can be comfortably and easily inserted into the passageway from which the sample will be taken. Additionally, the internal diameter of the internal bore 16 is similar to that of the internal diameter of other capnographic sampling systems which are in common use, thus ensuring a smooth flow of the sampled gas from the sampling catheter to the sampling system.

Sampling catheter 11 preferably includes a sealed and rounded end 18, sealing sampling portion 12. This structure facilitates insertion of sampling catheter 11 without scratching the tissues of the internal walls of the subject's passageways.

A top end 20 of the sampling catheter 11 is preferably fitted with a connector 22, which is adapted for attaching the sampling catheter 11 to a capnographic gas analyzer 24, such as Microcap® commercially available from Oridion Medical LTD. of Jerusalem, Israel, typically by means of standard sampling tubing.

A T-piece arrangement 26 may also be preferably provided at the top end 20 of the sampling catheter 11, to facilitate the pumping out of any liquids which may accumulate within the sampling catheter 11. T-piece arrangement 26 preferably enables connection of the catheter to a pumping system 28.

When using the sampling catheter 11, it is inserted into the nasal or oral orifice of a subject, and is pushed in preferably until the sampling portion 12 is located at least at the posterior pharynx of the subject, or further down the respiratory tract. At this location of the sampling catheter 11, the sampling is not sensitive to changes in the exhaled breath concentrations resulting from alternating oral and nasal breathing, and therefore the sampling is more accurate.

Preferably, longitudinal markings 30 are provided on an outer wall of the sampling catheter 11, allowing medical personnel inserting the catheter to determine the depth of catheter penetration.

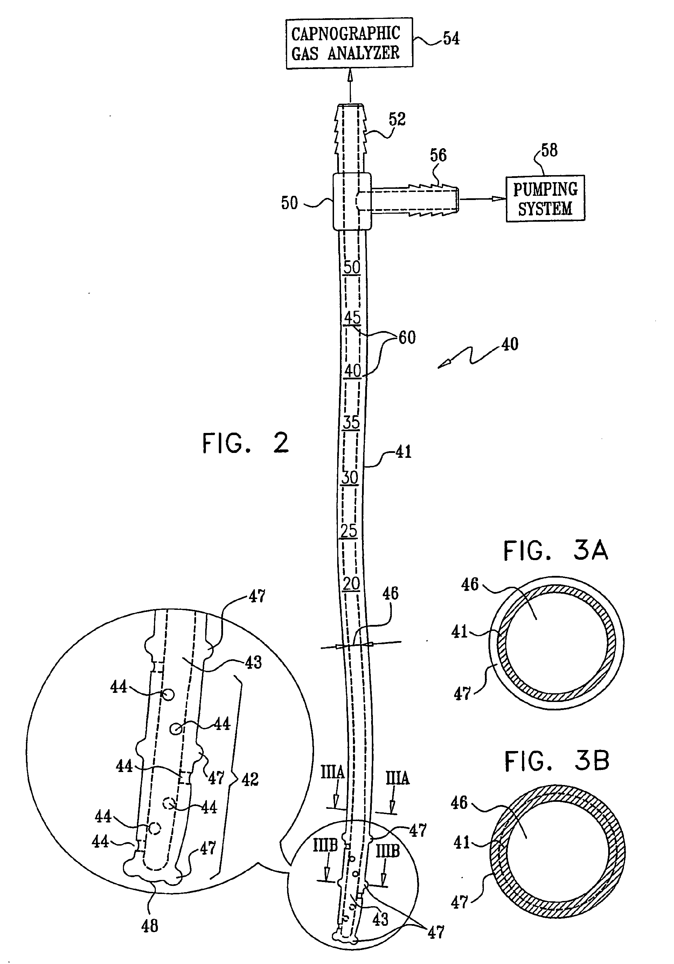

Reference is now made to Fig. 2, which is a schematic illustration of a capnographic sampling system constructed and operative in accordance with another preferred embodiment of the present invention and to Figs. 3A and 3B, which are sectional illustrations taken along respective section lines IIIA-IIIA and IIIB-IIIB thereof.

Capnographic system 40 includes a sampling catheter 41 having a sampling portion 42, which is preferably located at a lower end 43 of sampling catheter 41. A number of sampling holes 44 are formed in the wall of the sampling catheter 41, preferably within the sampling portion 42. Sampling catheter 41 additionally includes an internal bore 46, forming a passageway for the sampled fluid.

Sampling holes 44 are preferably arranged at different longitudinal positions along the length of sampling catheter 41 and at different circumferential positions therearound. A preferred pattern for the location of the sampling holes 44 is in the form of a helix. Additionally, at least two circumferential protrusions 47 are preferably formed on sampling catheter 41 at two restricting ends of sampling portion 42. More preferably, an additional circumferential protrusion 47 is formed in the middle of sampling portion 42, between sampling holes 44. The circumferential protrusions 47 distance the sampling holes 44 from a wall of the passageway into which the sampling catheter 41 has been inserted, such that even in the case of engagement between the sampling catheter 41 and the walls of the passageway all the sampling holes 44 remain open.

Additionally, the use of several sampling holes 44 ensures that even if a few of the sampling holes 44 are in contact with liquids, the gas sample will continue to flow through the open holes. This is due to the fact that the relatively small sampling holes have a greater resistance to liquids than to the sampled breath. Preferably, the gas sampling catheter 41 is formed of a hydrophobic material, and is therefore less prone to blockage of the sampling holes 44.

The diameter of the sampling holes 44 and the longitudinal distance between them are preferably determined according to the width of internal bore 46 and to the flow rate within the passageway from which the sample is taken. According to one preferred configuration of the sampling catheter 41, five sampling holes 44, each of a diameter in the range of 0.3 - 0.8mm are disposed within a 4cm-long sampling portion 42. Alternatively, a longer sampling portion 42 may be provided.

The sampling catheter 41 preferably has a narrow overall diameter and a narrow internal bore 46. For adult-sized use, the internal bore 46 is preferably of the order of 1.0mm, and for pediatric use, even less. The use of a catheter with such a narrow diameter is advantageous as it is readily filled by the sampled breath creating a faster response time. Furthermore, it can be comfortably and easily inserted into the passageway from which the sample will be taken. Additionally, the internal diameter of the internal bore 46 is similar to that of the internal diameter of other capnographic sampling systems which are in common use, thus ensuring a smooth flow of the sampled gas from the sampling catheter to the sampling system.

Sampling catheter 41 preferably includes a sealed and rounded end 48, sealing sampling portion 42. This structure facilitates insertion of sampling catheter 41 without scratching the tissues of the internal walls of the subject's passageways.

A top end 50 of the sampling catheter 41 is preferably fitted with a connector 52, which is adapted for attaching the sampling catheter 41 to a capnographic gas analyzer 54, such as Microcap® commercially available from Oridion Medical LTD. of Jerusalem, Israel, typically by means of standard sampling tubing.

A T-piece arrangement 56 may also be preferably provided at the top end 50 of the sampling catheter 41, to facilitate the pumping out of any liquids which may accumulate within the sampling catheter 41. T-piece arrangement 56 preferably enables connection of the catheter to a pumping system 58.

When using the sampling catheter 41, it is inserted into the nasal or oral orifice of a subject, and is pushed in preferably until the sampling portion 42 is located at least at the posterior pharynx of the subject, or further down the respiratory tract. At this location of the sampling catheter 41, the sampling is not sensitive to changes in the exhaled breath concentrations resulting from alternating oral and nasal breathing, and therefore the sample is more accurate.

Preferably, longitudinal markings 60 are provided on an outer wall of the sampling catheter 41, allowing medical personnel inserting the catheter to determine the depth of catheter penetration.

An essential difference between the embodiment of Fig. 1 and that of Figs. 2 - 3B is that the sampling holes on the sampling catheter cannot be blocked by engagement with the walls of the passageway into which the catheter is inserted due to the circumferential protrusions distancing the sampling holes from the passageway wall.

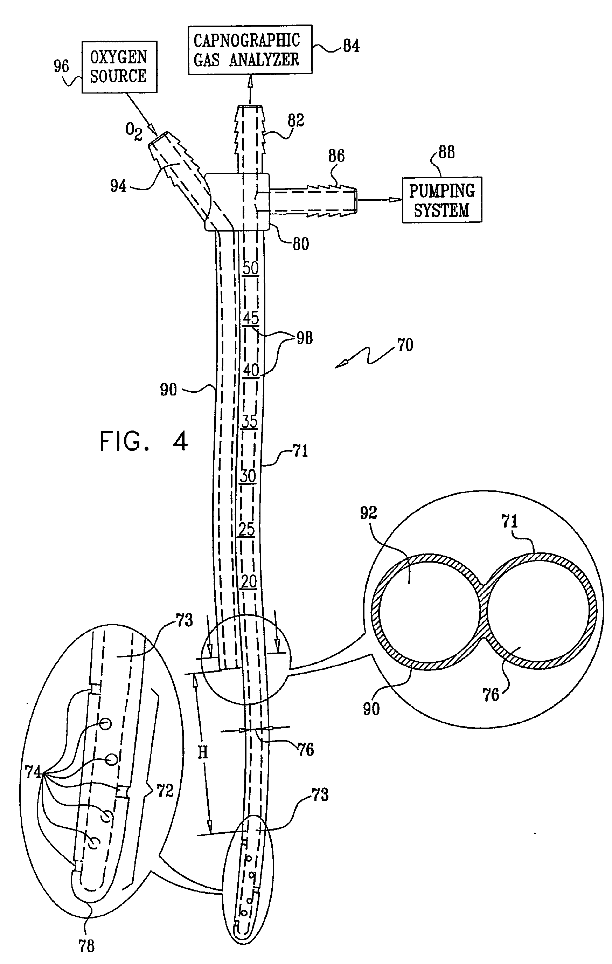

Reference is now made to Fig. 4, which is a schematic illustration of a capnographic sampling system constructed and operative in accordance with yet another preferred embodiment of the present invention.

Capnographic system 70 includes a sampling catheter 71 having a sampling portion 72, which is preferably located at a lower end 73 of sampling catheter 71. A number of sampling holes 74 are formed in the wall of the sampling catheter 71, preferably within the sampling portion 72. Sampling catheter 71 additionally includes an internal bore 76, forming a passageway for the sampled fluid.

Sampling holes 74 are preferably arranged at different longitudinal positions along the length of sampling catheter 71 and at different circumferential positions therearound. A preferred pattern for the location of the sampling holes 74 is in the form of a helix. This structure ensures that at least some of the sampling holes 74 remain open, even when one side of the catheter circumference is in contact with the passageway into which the sampling catheter 71 has been inserted.

Additionally, the use of several sampling holes 74 ensures that even if a few of the sampling holes 74 are in contact with liquids, the gas sample will continue to flow through the open holes. This is due to the fact that the relatively small sampling holes have a greater resistance to liquids than to the sampled breath. Preferably, the gas sampling catheter 71 is formed of a hydrophobic material, and is therefore less prone to blockage of the sampling holes 74.

The diameter of the sampling holes 74 and the longitudinal distance between them are preferably determined according to the width of internal bore 76 and to the flow rate within the passageway from which the sample is taken. According to one preferred configuration of the sampling catheter 71, five sampling holes 74, each of a diameter in the range of 0.3 - 0.8mm are disposed within a 4cm-long sampling portion 72. Alternatively, a longer sampling portion 72 may be provided.

The sampling catheter 71 preferably has a narrow overall diameter and a narrow internal bore 76. For adult-sized use, the internal bore 76 is preferably of the order of 1.0mm, and for pediatric use, even less. The use of a catheter with such a narrow diameter is advantageous as it is readily filled by the sampled breath, creating a faster response time. Furthermore, it can be comfortably and easily inserted into the passageway from which the sample will be taken. Additionally, the internal diameter of the internal bore 76 is similar to that of the internal diameter of other capnographic sampling systems which are in common use, thus ensuring a smooth flow of the sampled gas from the sampling catheter to the sampling system.

Sampling catheter 71 preferably includes a sealed and rounded end 78, sealing sampling portion 72. This structure facilitates insertion of sampling catheter 71 without scratching the tissues of the internal walls of the subject's passageways.

A top end 80 of the sampling catheter 71 is preferably fitted with a connector 82, which is adapted for attaching the sampling catheter 71 to a capnographic gas analyzer 84, such as Microcap® commercially available from Oridion Medical LTD. of Jerusalem, Israel, typically by means of standard sampling tubing.

A T-piece arrangement 86 may also be preferably provided at the top end 80 of the sampling catheter 71, to facilitate the pumping out of any liquids which may accumulate within the sampling catheter 71. T-piece arrangement 86 preferably enables connection of the catheter to a pumping system 88.

Sampling catheter 71 is preferably integrally formed with an oxygen delivery tube 90, surrounding an internal lumen 92, thereby forming a double-lumen structure with sampling catheter 71 as shown in the enlarged portion in Fig. 4. One end of oxygen delivery tube 90 is fitted with a connector 94, adapted to connect the oxygen delivery tube 90 to an oxygen source 96. Preferably, oxygen delivery tube 90 terminates at a point slightly higher than the beginning of sampling portion 72, in order to prevent dilution of sampled breath during exhalation by oxygen emitted from the oxygen delivery tube 90. Typically and preferably, the distance between the beginning of sampling portion 72 and the end of oxygen delivery tube 90, indicated by the letter H, is 2 or more centimeters.

When using the sampling catheter 71, it is inserted into the nasal or oral orifice of a subject, and is pushed in preferably until the sampling portion 72 is located at least at the posterior pharynx of the subject, or further down the respiratory tract. At this location of the sampling catheter 71, the sampling is not sensitive to changes in the exhaled breath concentrations resulting from alternating oral and nasal breathing, and thereby the sampling is more accurate. Moreover, this location of the sampling catheter 71 will ensure sufficient oxygen supply to the subject's respiratory tract through oxygen delivery tube 90, which is also located at or near the posterior pharynx of the subject.

Preferably, longitudinal markings 98 are provided on an outer wall of the sampling catheter 71, allowing medical personnel inserting the catheter to determine the depth of catheter penetration.

An essential advantage of the embodiment of Fig. 4 is that the design is specifically suitable for sedated yet spontaneously breathing patients.

It is appreciated by persons skilled in the art that the present invention is not limited by what has been particularly shown and described hereinabove. Rather the scope of the present invention includes both combinations and subcombinations of various features described hereinabove as well as variations and modifications thereto which would occur to a person of skill in the art upon reading the above description and which are not in the prior art.

## Claims

1. A breath sampling catheter (11) comprising a catheter tube being formed with a plurality of exhaled breath sampling holes (14), **characterized in that** said plurality of exhaled breath sampling holes (14) is disposed at different circumferential positions around said gas sampling catheter (11).

2. A breath sampling catheter (11) according to claim 1, wherein different ones of said plurality of exhaled breath sampling holes (14) are disposed at different longitudinal positions along said catheter tube (11).

3. A breath sampling catheter (11) according to any of claims 1 to 2 and wherein said plurality of exhaled breath sampling holes (14) are disposed in a generally helical pattern.

4. A breath sampling catheter (11) according to any of claims 1 to 3 and wherein one end (18) of said catheter tube is sealed.

5. A breath sampling catheter (11) according to any of claims 1 to 4 and wherein said catheter has at least one circumferential protrusion (47) formed therearound.

6. A breath sampling catheter (11) according to any of claims 1 to 5 also comprising a suction element (58), configured to remove fluid from said gas sampling catheter (11).

7. A breath sampling catheter (11) according to any of claims 1 to 6 and also comprising:
an oxygen delivery tube (90) mounted onto said gas sampling catheter (11), thereby forming a multi-lumen tube therewith; and
an oxygen source (96) connected to said oxygen delivery tube (90) for supply of oxygen thereto.

8. A system for analyzing the exhaled breath of a subject, comprising:
a gas analyzer (24), and a gas sampling catheter (11) according to any of claims 1-7, wherein said gas analyzer (24) is sensitive to at least one component of said exhaled breath; and wherein said gas sampling catheter (11) has a first end (20) and a second end (18), said first end being in fluid flow communication with said gas analyzer (24).

9. A system according to claim 8 and wherein said first end (20) of said gas sampling catheter (11) is directly connected to said gas analyzer (24), thereby forming said fluid flow communication therebetween.

10. A system according to claims 8 to 9 and wherein said first end (20) of said gas sampling catheter (11) is connected to said gas analyzer by means of a breath connector (22).

11. A system according to claims 8 to 10 and wherein said gas sampling catheter (11) is fitted with a suction port (56), said suction port (56) being adapted to connect to said suction element (58).

## Patentansprüche

1. Atemprobenahmekatheter (11), aufweisend einen Katheterschlauch, der mit einer Mehrzahl von Probenahmeöffnungen (14) für ausgestoßenen Atem ausgebildet ist, **dadurch gekennzeichnet, dass** die Mehrzahl der Probenahmeöffnungen (14) für ausgestoßenen Atem an unterschiedlichen Positionen am Umfang des Gasprobenahmekatheters (11) angeordnet ist.

2. Atemprobenahmekatheter (11) nach Anspruch 1, wobei unterschiedliche der Mehrzahl von Probenahmeöffnungen (14) für ausgestoßenen Atem an unterschiedlichen Positionen in der Längsrichtung entlang des Katheterschlauchs (11) angeordnet sind.

3. Atemprobenahmekatheter (11) nach einem der Ansprüche 1 bis 2, und wobei die Mehrzahl von Probenahmeöffnungen (14) für ausgestoßenen Atem in einem im Wesentlichen spiralförmigen Muster angeordnet ist.

4. Atemprobenahmekatheter (11) nach einem der Ansprüche 1 bis 3, und wobei ein Ende (18) des Katheterschlauchs abgedichtet ist.

5. Atemprobenahmekatheter (11) nach einem der Ansprüche 1 bis 4, und wobei der Katheter mindestens einen Vorsprung (47) am Umfang hat, der darum gebildet ist.

6. Atemprobenahmekatheter (11) nach einem der Ansprüche 1 bis 5, auch aufweisend ein Saugelement (58), das konfiguriert ist, Fluid aus dem Gasprobenahmekatheter (11) zu entfernen.

7. Atemprobenahmekatheter (11) nach einem der Ansprüche 1 bis 6, und auch aufweisend:
einen Sauerstoffzufuhrschlauch (90), der am Gasprobenahmekatheter (11) befestigt ist, wodurch damit ein mehrlumiger Schlauch gebildet wird; und
eine Sauerstoffquelle (96), die mit dem Sauerstoffzufuhrschlauch (90) verbunden ist, um ihn mit Sauerstoff zu versorgen.

8. System zum Analysieren des ausgestoßenen Atems eines Probanden, aufweisend:
einen Gasanalysator (24) und einen Gasprobenahmekatheter (11) nach einem der Ansprüche 1 bis 7, wobei der Gasanalysator (24) für mindestens eine Komponente des ausgestoßenen Atems empfindlich ist, und wobei der Gasprobenahmekatheter (11) ein erstes Ende (20) und ein zweites Ende (18) hat, wobei das erste Ende mit dem Gasanalysator (24) in Fluidströmungskommunikation steht.

9. System nach Anspruch 8, und wobei das erste Ende (20) des Gasprobenahmekatheters (11) direkt mit dem Gasanalysator (24) verbunden ist, wodurch die Fluidströmungskommunikation dazwischen gebildet wird.

10. System nach Anspruch 8 bis 9, und wobei das erste Ende (20) des Gasprobenahmekatheters (11) über ein Atemverbindungsstück (22) mit dem Gasanalysator verbunden ist.

11. System nach Anspruch 8 bis 10, und wobei der Gasprobenahmekatheter (11) mit einem Sauganschluss (56) ausgestattet ist, wobei der Sauganschluss (56) ausgelegt ist, mit dem Saugelement (58) verbunden zu werden.

## Revendications

1. Cathéter d'échantillonnage capnographique (11) comprenant un tube de cathéter constitué d'une pluralité d'orifices d'échantillonnage de la respiration exhalée (14), **caractérisé en ce que** ladite pluralité d'orifices d'échantillonnage de la respiration exhalée (14) est disposée à différents emplacements circonférentiels autour dudit cathéter d'échantillonnage de gaz (11).

2. Cathéter d'échantillonnage capnographique (11), selon la revendication 1, dans lequel différents trous parmi la pluralité d'orifices d'échantillonnage de la respiration exhalée (14) sont disposés à différents emplacements longitudinaux le long dudit tube de cathéter (11).

3. Cathéter d'échantillonnage capnographique (11), selon l'une quelconque des revendications 1 à 2 dans lequel ladite pluralité d'orifices d'échantillonnage de la respiration exhalée (14) est disposée selon un motif généralement hélicoïdal.

4. Cathéter d'échantillonnage capnographique (11), selon l'une quelconque des revendications 1 à 3, dans lequel une extrémité (18) dudit tube de cathéter est scellée.

5. Cathéter d'échantillonnage capnographique (11), selon l'une quelconque des revendications 1 à 4, dans lequel ledit cathéter a au moins une saillie circonférentielle (47) formée autour de lui.

6. Cathéter d'échantillonnage capnographique (11), selon l'une quelconque des revendications 1 à 5, comprenant également un élément d'aspiration (58) configuré de façon à éliminer le fluide provenant dudit cathéter d'échantillonnage de gaz (11).

7. Cathéter d'échantillonnage capnographique (11), selon l'une quelconque des revendications 1 à 6, et comprenant également :
un tube de distribution d'oxygène (90) monté sur ledit cathéter d'échantillonnage de gaz (11), en formant ainsi un tube multi-lumière avec lui ; et
une source d'oxygène (96) raccordée audit tube de distribution d'oxygène (90) pour permettre l'alimentation en oxygène.

8. Système pour analyser la respiration exhalée d'un sujet, comprenant :
un analyseur de gaz (24), un cathéter d'échantillonnage de gaz (11) selon l'une quelconque des revendications 1-7, dans lequel ledit analyseur de gaz (24) est sensible à au moins un composant de ladite respiration exhalée ; et dans lequel ledit cathéter d'échantillonnage de gaz (11) a une première extrémité (20) et une seconde extrémité (18), ladite première extrémité étant en communication fluidique avec ledit analyseur de gaz (24).

9. Système selon la revendication 8, et dans lequel ladite première extrémité (20) dudit cathéter d'échantillonnage de gaz (11) est directement raccordée audit analyseur de gaz (24), en formant ainsi ladite communication fluidique entre eux.

10. Système selon les revendications 8 à 9, et dans lequel ladite première extrémité (20) dudit cathéter d'échantillonnage de gaz (11) est raccordée audit analyseur de gaz, au moyen d'un connecteur de respiration (22).

11. Système selon les revendications 8 à 10, et dans lequel ledit cathéter d'échantillonnage de gaz (11) est équipé d'un orifice d'aspiration (56), ledit orifice d'aspiration (56) étant adapté pour connecter ledit élément d'aspiration (58).
